(19) Europäisches Patentamt
European Patent Office
Office européen des brevets

(11) Veröffentlichungsnummer : **0 409 368 B1**

(12) # EUROPÄISCHE PATENTSCHRIFT

(45) Veröffentlichungstag der Patentschrift :
**11.11.92 Patentblatt 92/46**

(51) Int. Cl.$^5$ : **C07D 239/60,** A01N 43/54

(21) Anmeldenummer : **90250184.0**

(22) Anmeldetag : **19.07.90**

(54) **Substituierte alfa-Pyrimidinyloxy-carbonsäurederivate, Verfahren zu ihrer Herstellung und ihre Verwendung als Mittel mit herbizider, fungizider und pflanzenwachstumsregulierender Wirkung.**

Die Akte enthält technische Angaben, die nach dem Eingang der Anmeldung eingereicht wurden und die nicht in dieser Patentschrift enthalten sind.

(30) Priorität : **19.07.89 DE 3924259**
**22.03.90 DE 4009481**

(43) Veröffentlichungstag der Anmeldung :
**23.01.91 Patentblatt 91/04**

(45) Bekanntmachung des Hinweises auf die Patenterteilung :
**11.11.92 Patentblatt 92/46**

(84) Benannte Vertragsstaaten :
**AT BE CH DE DK ES FR GB GR IT LI LU NL SE**

(56) Entgegenhaltungen :
**EP-A- 0 223 406**
**EP-A- 0 347 811**
**EP-A- 0 400 741**

(73) Patentinhaber : **SCHERING AKTIENGESELLSCHAFT Berlin und Bergkamen**
**Müllerstrasse 170/178 Postfach 65 03 11**
**W-1000 Berlin 65 (DE)**

(72) Erfinder : **Wegner, Peter, Dr.**
**Münchener Strasse 3**
**W-1000 Berlin 28 (DE)**
Erfinder : **Harde, Christoph, Dr.**
**Südwestkorso 61**
**W-1000 Berlin 41 (DE)**
Erfinder : **Nordhoff, Erhard, Dr.**
**Markelstrasse 45**
**W-1000 Berlin 41 (DE)**
Erfinder : **Krüger, Anita, Dr.**
**Schluchseestrasse 65**
**W-1000 Berlin 28 (DE)**
Erfinder : **Krüger, Gabriele, Dr.**
**Bachstrasse 3**
**W-1000 Berlin 21 (DE)**
Erfinder : **Tarara, Gerhard, Dr.**
**Sprengelstrasse 36**
**W-1000 Berlin 65 (DE)**
Erfinder : **Heinrich, Nikolaus, Dr.**
**Berliner Strasse 14a**
**W-1000 Berlin 37 (DE)**
Erfinder : **Rees, Richard, Dr.**
**Speerweg 8**
**W-1000 Berlin 28 (DE)**
Erfinder : **Johann, Gerhard, Dr.**
**Hermsdorfer Damm 147**
**W-1000 Berlin 28 (DE)**
Erfinder : **Kötter, Clemens, Dr.**
**Laurinsteig 26**
**W-1000 Berlin 28 (DE)**

EP 0 409 368 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue substituierte α-Pyrimidinyloxy carbonsäurederivate, Verfahren zu ihrer Herstellung und ihre Verwendung als Mittel mit herbizider, fungizider und pflanzenwachstumsregulierender Wirkung.

Es ist bereits bekannt, daß Pyrimidinderivate eine herbizide Wirkung besitzen (EP-Anmeldungen 0 223 406, 0 249 707, 0 249 708, 0 287 072 und 0 287 079). Häufig ist jedoch die Herbizidwirkung der bekannten Verbindungen nicht ausreichend, beziehungsweise es treten bei entsprechender Herbizidwirkung Selektivitätsprobleme in landwirtschaftlichen Hauptkulturen auf.

In der EP-A-0 347 811 werden Verbindungen der Formel

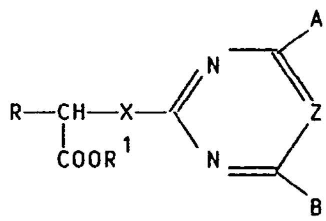

beansprucht, in der, inter alia, X gleich Sauerstoff, A und B gleich Alkoxy, Z gleich CH oder N, $R^1$ gleich Wasserstoff oder andere Estergruppen und R gleich $CR^2R^3R^4$ ist, wobei $R^2$ und $R^4$ gemeinsam einen Ring bilden können oder einzeln Alkyl sind, und $R^3$, inter alia, gleich Wasserstoff, Halogen, Alkyl oder Haloalkyl bedeuten. In dieser Drucktschrift wird kein Beispiel einer Verbindung gegeben, bei dem $R^3$ Halogen ist, wenn $R^3$ und $R^4$ gemeinsam einen Ring bilden oder beide Alkyl bedeuten. Des weiteren wird auch kein Beispiel gegeben, bei dem $R^3$ gleich Fluor bedeutet.

Auch in der EP-A-0 400 741 werden Pyrimidinyloxycarbonsäuren beschrieben, die jedoch in α-Stellung keinen durch Fluor substituierten Alkyl- oder Cycloalkylrest aufweisen.

In EP-A 0 409 368 werden Pyrimidinyloxy- und thio-benzoesäurederivate beschrieben.

Aufgabe der vorliegenden Erfindung ist die Bereitstellung von neuen Verbindungen, die diese Nachteile nicht aufweisen und die in ihren biologischen Eigenschaften den bisher bekannten Verbindungen überlegen sind.

Es wurde nun gefunden, daß substituierte α-Pyrimidinyloxy-carbonsäurederivate der allgemeinen Formel I

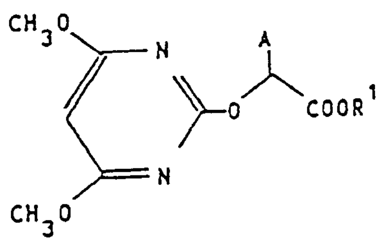

(I)

in der

A einen in 1-stellung durch Fluor substituierten $C_1$-$C_6$-Alkyl-oder $C_3$-$C_{60}$-Cycloalkylrest oder den α-Methyl-α-fluorbenzyl-rest

$R^1$ ein Wasserstoffatom, einen $C_1$-$C_4$-Alkylrest oder den Benzylrest bedeuten, und deren optische Isomere eine interessante herbizide, fungizide und pflanzenwachstumstegulierende Wirkung zeigen.

Die erfindungsgemäßen Verbindungen der allgemeinen Formel I können zum Beispiel hergestellt werden, indem man

A) Verbindungen der allgemeinen Formel II

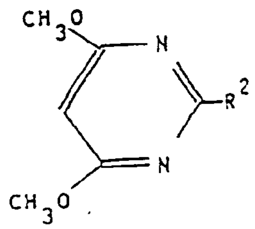

(II)

in der $R^2$, für ein Halogenatom, eine Alkylsulfonylgruppe oder eine Phenylsulfonylgruppe steht, mit Verbindungen der allgemeinen Formel III

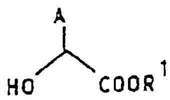

(III)

in der A, und $R^1$ die unter der allgemeinen Formel I genannten Bedeutungen haben, in einem geeigneten Lösungsmittel in Gegenwart einer geeigneten Base umsetzt, oder

B) Verbindungen der allgemeinen Formel IV

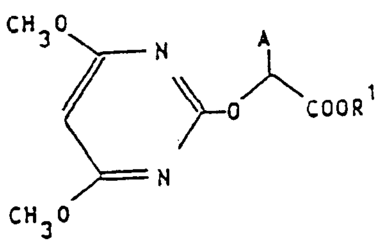

(IV)

in der A und $R^1$ die unter der allgemeinen Formel I angegebenen Bedeutungen haben, in einem geeigneten polaren Lösungsmittel mit einer Alkalimetallbase oder einen Erdalkalimetallbase zu Verbindungen der allgemeinen Formel V

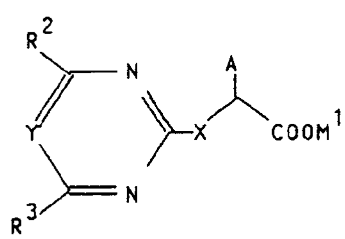

(V)

in der A, $R^2$, $R^3$, X und Y die unter der allgemeinen Formel I angegebenen Bedeutungen haben und $M^1$ für ein Alkalimetallatom oder ein Äquivalent eines Erdalkalimetallatoms steht, umsetzt, und auschließend mit einer geeigneten Säure in einem geeigneten Lösungsmittel zu Verbindungen der allgemeinen Formel VI

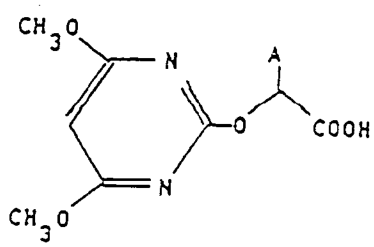

(VI)

in der A die unter der allgemeinen Formel I angegebene Bedeutung hat umsetzt, oder

C) Verbindungen der allgemeinen Formel VIII

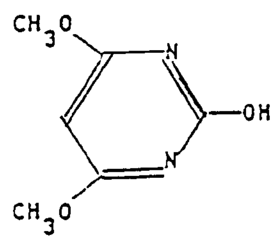

(VIII)

mit einer Verbindung der allgemeinen Formel IX

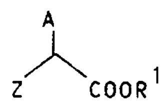

(IX)

in der A und $R^1$ die unter der allgemeinen Formel I angegebenen Bedeutungen haben und Z für ein Halogenatom oder eine Alkylsulfonyloxygruppe steht, in Anwesenheit eines geeigneten Lösungsmittels und einer geeigneten Base umsetzt, oder

D) eine Verbindung der allgemeinen Formel X

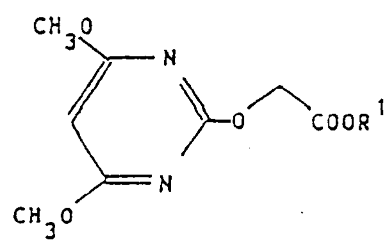

(X)

in der $R^1$ die unter der allgemeinen Formel I angegebene Bedeutung hat, mit einer Verbindung der allgemeinen Formel XI

$$A - R^2 \qquad (XI)$$

in der A die unter der allgemeinen Formel I angegebenen Bedeutungen hat und $R^2$ für ein Halogenatom, eine Alkylsulfonyloxygruppe oder Phenylsulfonyloxygruppe in einem geeigneten Lösungsmittel in Anwesenheit einer geeigneten Base umsetzt.

Die einzelnen Verfahrensvarianten werden vorzugsweise in Gegenwart eines Verdünnungsmittels durchgeführt. Zu diesem Zweck können sämtliche gegenüber den verwendeten Reagenzien inerte Lösungsmittel verwendet werden.

Beispiele für solche Lösungsmittel beziehungsweise Verdünnungsmittel sind Wasser, aliphatische, alicyclische und aromatische Kohlenwasserstoffe, die jeweils gegebenenfalls chloriert sein können, wie zum Beispiel

Hexan, Cyclohexan, Petrolether, Ligroin, Benzol, Toluol, Xylol, Methylenchlorid, Chloroform, Kohlenstofftetrachlorid, Ethylenchlorid und Trichlorethylen, Ether, wie zum Beispiel Diisopropylether, Dibutylether, Propylenoxid, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum Beispiel Methanol, Ethanol, Isopropanol, Butanol und Ethylenglycol, Ester, wie zum Beispiel Ethylacetat und Amylacetat, Säureamide, wie zum Beispiel Dimethylformamid und Dimethylacetamid, Sulfoxide und Sulfone, wie zum Beispiel Dimethylsulfoxid und Sulfolan, und Basen, wie zum Beispiel Pyridin.

Die Gegenwart eines Reaktionskatalysators kann von Vorteil sein. Als Katalysatoren sind Kaliumjodid und Oniumverbindungen geeignet, wie quaternäre Ammonium-, Phosphonium- und Arsoniumverbindungen sowie Sulfoniumverbindungen. Ebenfalls geeignet sind Polyglycolether, insbesondere cyclische, wie zum Beispiel 18-Krone-6, und tertiäre Amine, wie zum Beispiel Tributylamin. Bevorzugte Verbindungen sind quaternäre Ammoniumverbindungen, wie zum Beispiel Benzyltriethylammoniumchlorid und Tetrabutylammoniumbromid.

Die Reaktionen lassen sich bei dem Druck der Umgebung durchführen, wenngleich sie auch bei erhöhtem beziehungsweise vermindertem Druck durchgeführt werden können.

Die Verfahrensvariante A) wird bevorzugt in aromatischen Kohlenwasserstoffen, wie Benzol, Toluol oder Xylol, halogenierte Kohlenwasserstoffe, wie Methylenchlorid oder Chloroform, Alkoholen, wie Methanol, Ethanol oder Isopropanol, Ethern, wie Ethylether, Isopropylether, Tetrahydrofuran oder 1,4-Dioxan, Ketonen, wie Aceton oder Methylethylketon, Ester, wie Methylacetat oder Ethylacetat, polaren aprotischen Lösungsmitteln, wie Dimethyl formamid, Dimethylacetatamid oder Dimethylsulfoxid, und in anderen Lösungsmitteln, wie Acetonitril oder Wasser, durchgeführt.

Als Base kann ein Alkalimetall, wie Natrium oder Kalium, ein Alkali- oder Erdalkalimetallhydrid, wie Natriumhydrid, Kaliumhydrid oder Calciumhydrid, ein Karbonat, wie Natriumcarbonat oder Kaliumcarbonat oder ein Metallhydroxid, wie Natriumhydroxid oder Kaliumhydroxid verwendet werden.

Die Reaktion wird vorzugsweise in einem Temperaturbereich zwischen Raumtemperatur und dem Siedepunkt des Lösungsmittels oder Lösungsmittelgemisches durchgeführt. Die Reaktionszeit beträgt 1 bis 24 Stunden.

Die Reaktion kann aber auch in Abwesenheit eines Lösungsmittels, in einem Temperaturbereich von 120 bis 160°C, unter Verwendung eines Alkalimetallcarbonats, wie wasserfreies Kaliumcarbonat, durchgeführt werden.

Zur Herstellung der bei dieser Verfahrensvariante benötigten α-Hydroxy-beziehungsweise α-Mercaptocarbonsäurederivate können zum Beispiel folgende Literaturverfahren verwendet werden: J. Org. Chem. 33, 2565 [1968]; J. Amer. Chem. Soc. 95, 7146 [1973]; J. Chem. Soc. 1957, 3262; J. Org. Chem. 33, 1831 [1968]; Can. J. Chem. 60 [1982] 2707; Bull. Soc. Chim. Fr. 1969, 2721.

Die Verfahrensvariante B) wird bevorzugt in Alkoholen, wie Methanol, Ethanol oder Isopropanol, in Ketonen, wie Aceton oder Methylketonen, in Wasser oder in einer Mischung aus Wasser und einem polaren Lösungsmittel durchgeführt.

Als Base kann ein Carbonat, wie Natriumcarbonat, Kaliumkarbonat oder Calciumcarbonat und ein Metallhydroxid, wie Natriumhydroxid oder Kaliumhydroxid, verwendet werden.

Der Temperaturbereich der Reaktionen liegt zwischen Raumtemperatur und dem Siedepunkt des jeweiligen Lösungsmittels oder Lösungsmittelgemisches. Die Reaktionzeit beträgt 0,5 bis 36 Stunden.

Falls $R^1$ in der allgemeinen Formel IV eine Benzylgruppe bedeutet, kann eine Verbindung der allgemeinen Formel VI auch durch katalytische Reduktion (Hydrierung) erhalten werden.

Für die Verfahrensvariante C) können als Lösungsmittel Kohlenwasserstoffe, wie Benzol, Toluol oder Xylol, halogenierte Kohlenwasserstoffe, wie Methylenchlorid oder Chloroform, Ether, wie Ethylether, Isopropylether, Tetrahydrofuran oder 1,4-Dioxan, Ketone, wie Aceton oder Methylethylketon, Ester, wie Methylacetat oder Ethylacetat, aprotische polare Lösungsmittel, wie Dimethylformamid, Dimethylacetamid oder Dimethylsulfoxid und andere Lösungsmittel, wie Acetonitril, verwendet werden.

Als Basen können Alkalimetalle, wie Natrium oder Kalium, Alkalimetall- oder Erdalkalimetallhydride, wie Natriumhydrid, Kaliumhyhydrid oder Calciumhydrid, Carbonate, wie Natriumcarbonat, Kaliumcarbonat, Natriumhydrogencarbonat oder Kaliumhydrogencarbonat, oder Metallhydroxide, wie Natriumhydroxid oder Kaliumhydroxid, verwendet werden.

Der Temperaturbereich der Reaktion liegt zwischen Raumtemperatur und dem Siedepunkt des jeweiligen Lösungsmittel oder Lösungsmittelgemisches. Die Reaktionszeit beträgt 5 Minuten bis 24 Stunden.

Die Verfahrensvariante D) wird vorzugsweise in aprotischen Lösungsmitteln, wie Benzol, Toluol, Xylol, Tetrahydrofuran, Diethylether, Hexan, Dimethylformamid oder Dimethylsulfoxid, durchgeführt. Zur Deprotonierung der Verbindungen der allgemeinen Formel X können Basen, wie Natriumhydrid, Kaliumtertiärbutylat oder Lithiumdiisopropylamid verwendet werden.

Die Reaktionstemperatur liegt zwischen -78°C und der Siedetemperatur des jeweiligen Lösungsmittels

oder Lösungsmittelgemisches. Die Reaktionszeit beträgt 0,5 bis 24 Stunden.

Die Verbindungen der allgemeinen Formel X sind in der Literatur beschrieben oder können nach in der Literatur angebenen Verfahren hergestellt werden [Khim.-Farm. Zh. 16 (8), 931-4 [1982]; Ukr. Khim. Zh. (Russ.Ed.) 49 (11), 1205-8 [1983]; Fizol. Akt. Veshchestva, 18, 75-9 [1986]; USSR-PS 791746).

Die nach den oben genannten Verfahren hergestellten erfindungsgemäßen Verbindungen können nach den üblichen Verfahren aus dem Reaktionsgemisch isoliert werden, beispielsweise durch Abdestillieren des eingesetzten Lösungsmittels bei normalem oder vermindertem Druck, durch Ausfällen mit Wasser oder durch Extraktion.

Ein erhöhter Reinheitsgrad kann in der Regel durch säulenchromatographische Aufreinigung sowie durch fraktionierte Destillation oder Kristallisation erhalten werden.

Die erfindungsgemäßen Verbindungen stellen in der Regel farb- und geruchlose Flüssigkeiten sowie Kristalle dar, die löslich in Wasser, wenig löslich in aliphatischen Kohlenwasserstoffen, wie Petrolether, Hexan, Pentan und Cyclohexan, gut löslich in halogenierten Kohlenwasserstoffen, wie Chloroform, Methylenchlorid und Tetrachlorkohlenstoff, aromatischen Kohlenwasserstoffen, wie Benzol, Toluol und Xylol, Ethern, wie Diethylether, Tetrahydrofuran und Dioxan, Carbonsäurenitrilen, wie Acetonitril, Alkoholen, wie Methanol und Ethanol, Carbonsäureamiden, wie Dimethylformamid, und Sulfoxiden, wie Dimethylsulfoxid, sind.

Die erfindungsgemäßen Verbindungen zeigen eine gute herbizide Wirkung bei breitblättrigen Unkräutern und Gräsern. Ein selektiver Einsatz der erfindungsgemäßen Wirkstoffe ist in verschiedenen Kulturen möglich, zum Beispiel in Raps, Rüben, Sojabohnen, Baumwolle, Reis, Gerste, Weizen und anderen Getreidearten. Dabei sind einzelne Wirkstoffe als Selektivherbizide in Rüben, Baumwolle, Soja und Getreide besonders geeignet. Ebenso können die Verbindungen zur Unkrautbekämpfung in Dauerkulturen, wie zum Beispiel Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, und zur selektiven Unkrautbekämpfung in einjährigen Kulturen eingesetzt werden.

Die erfindungsgemäßen Verbindungen können zum Beispiel bei den folgenden Pflanzengattungen verwendet werden:

Dikotyle Unkräuter der Gattungen Sinapis, Lepidium, Galium, Stellaria, Matricaria, Anthemis, Galinsoga, Chenopodium, Brassica, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Lamium, Veronica, Abutilon, Datura, Viola, Galeopsis, Papaver, Centaurea und Chrysanthemum.

Monokotyle Unkräuter der Gattungen Avena, Alopecurus, Echinochloa, Setaria, Panicum, Digitaria, Poa, Eleusine, Brachiaria, Lolium, Bromus, Cyperus, Agropyron, Sagittaria, Monochoria, Fimbristylis, Eleocharis, Ischaemum und Apera.

Die Aufwandmengen schwanken je nach Anwendungsart im Vor- und Nachauflauf in Grenzen zwischen 0,001 bis 5 kg/ha.

Die erfindungsgemäßen Wirkstoffe können auch als Defoliant, Desiccant und als Krautabtötungsmittel verwendet werden. Sie beeinflussen auch das Pflanzenwachstum und können deshalb zur Wachstumsbeeinflussung von Kulturpflanzen eingesetzt werden. Einige dieser Wirkstoffe zeigen auch eine fungizide Wirkung.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden. Sofern eine Verbreiterung des Wirkungsspektrums beabsichtigt ist, können auch andere Herbizide zugesetzt werden. Beispielsweise eignen sich als herbizid wirksame Mischungspartner diejenigen Wirkstoffe, die in Weed Abstracts, Vol. 38, No. 3, 1989, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Zweckmäßig werden die gekennzeichneten Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe, wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

A) **Spritzpulver**

1.)

25 Gewichtsprozent Wirkstoff
60 Gewichtsprozent Kaolin
10 Gewichtsprozent Kieselsäure
5 Gewichtsprozent einer Mischung aus dem Calciumsalz der Ligninsulfonsäure und dem Natriumsalz des N-Methyl-N-oleyl-taurins

2.)

40 Gewichtsprozent Wirkstoff
25 Gewichtsprozent Tonmineralien
25 Gewichtsprozent Kieselsäure
10 Gewichtsprozent einer Mischung aus dem Calciumsalz der Ligninsulfonsäure und Alkylphenylpolyglycolethern

B) **Paste**

45 Gewichtsprozent Wirkstoff
5 Gewichtsprozent Natriumaluminiumsilikat
15 Gewichtsprozent Cetylpolyglycolether mit 8 Mol Ethylenoxid
2 Gewichtsprozent Spindelöl
10 Gewichtsprozent Polyethylenglycol
23 Gewichtsprozent Wasser

C) **Emulsionskonzentrat**

25 Gewichtsprozent Wirkstoff
15 Gewichtsprozent Cyclohexanon
55 Gewichtsprozent Xylol
5 Gewichtsprozent einer Mischung aus dem Calciumsalz der Dodecylbenzolsulfonsäure und Nonylphenylpolyoxyethylen

Die nachfolgenden Beispiele erläutern die Herstellung der erfindungsgemäßen Verbindungen:

Beispiel 1

**2-(4,6-Dimethoxy-2-pyrimidinyloxy)-3-fluor-3-methylbutansäuremethylester**

4 g (26,6 mmol) 3-Fluor-2-hydroxy-3-methylbutansäuremethylester und 5,81 g (26,6 mmol) 4,6-Dimethoxy-2-methylsulfonylpyrimidin werden in 75 ml Dimethylformamid bei 20°C gelöst und mit 1,84 g (13,3 mmol) Kaliumcarbonat versetzt. Man rührt die Suspension 5 Stunden bei 20°C und erwärmt danach eine Stunde auf 60°C. Das Reaktionsgemisch wird auf 150 ml Eiswasser gegeben und dreimal mit je 75 ml Essigester extrahiert. Die vereinigten Essigesterphasen werden mit Wasser gewaschen, über Magnesiumsulfat getrocknet, filtriert und eingeengt. Der so erhaltene Feststoff wird durch Säulenchromatographie an Kieselgel mit Hexan/Essigestergemischen gereinigt.
Ausbeute: 2,9 g = 37,7 % der Theorie
Fp.: 73-74°C

Beispiel 2

**2-(4,6-Dimethoxy-2-pyrimidinyloxy)-3-fluor-3-methylbutansäure**

2 g (6,9 mmol) 2-(4,6-Dimethoxy-2-pyrimidinyloxy)-3-fluor-3-methylbutansäuremethylester werden in einem Gemisch von 20 ml Wasser und 10 ml Methanol gelöst, mit 387 mg (6,9 mmol) Kaliumhydroxyd versetzt und 4 Stunden bei 50°C gerührt. Die Reaktionslösung wird auf 50 ml Wasser gegeben und mit 50 ml Essigester extrahiert. Danach säuert man die wäßrige Phase mit Salzsäure auf pH 2 an und extrahiert diese dreimal mit je 100 ml Essigester. Die vereinigten Essigesterphasen werden mit Wasser gewaschen, über Magnesiumsulfat getrocknet, filtriert und eingeengt.
Ausbeute: 1,0 g = 54,5 % der Theorie
Fp.: 107-108°C

In analoger Weise wurden auch die folgenden erfindungsgemäßen Verbindungen der allgemeinen Formel I hergestellt:

| Beispiel Nr. | A | $R^1$ | Physikalische konstante |
|---|---|---|---|
| 3 | $CF(CH_3)_2$ | $CH_2CH_3$ | Fp. : 73-74°C |
| 4 | $CF(CH_3)$-Phenyl | $CH_2CH_3$ | Fp. : 106°C |
| 5 | 1-Fluor-cyclohexyl | $CH_3$ | Fp. : 99-100°C |
| 6 | $CF(CH_3)$-Phenyl | H | Fp. : 135-136°C |
| 7 | 1-Fluor-cyclopentyl | $CH_3$ | Fp. : 89-90°C |
| 8 | 1-Fluor cyclohexyl | H | Fp. : 140-141°C |
| 9 | 1-Fluor-cyclopentyl | H | Fp. : 138-140°C |

**Beispiel A**

Im Gewächshaus wurden die aufgeführten Pflanzenspezies vor dem Auflaufen mit den aufgeführten Verbindungen in einer Aufwandmenge von 1,0 kg Wirkstoff/ha behandelt. Die Verbindungen wurden zu diesem Zweck als Emulsionen oder Suspensionen mit 500 Litern Wasser/ha gleichmäßig über den Boden versprüht. Hier zeigten drei Wochen nach der Behandlung die erfindungsgemäßen Verbindungen eine ausgezeichneter Wirkung gegen das Unkraut.
In der folgenden Tabelle bedeuten:
0 = keine Schädigung
1 = 1 - 24 % Schädigung
2 = 25 - 74 % Schädigung
3 = 75 - 89 % Schädigung
4 = 90 - 100 % Schädigung
AVEFA = Avena fatua
SETVI = Setaria viridis
CYPES = Cyperus esculentus
ABUTH = Abutilon theophrasti
IPOSS = Ipomoea purpurea

MATCH = Matricaria chamomilla
POLSS = Polygonum sp.
SOLSS = Solanum sp.
VERPE = Veronica persica
VIOSS = Viola sp.

| Erfindungsgemäße Verbindungen | AVEFA | SETVI | CYPES | ABUTH | IPOSS | MATCH | POLSS | SOLSS | VERPE | VIOSS |
|---|---|---|---|---|---|---|---|---|---|---|
| Beispiel 1 | 3 | 4 | 4 | 3 | 3 | 3 | - | - | - | - |
| Beispiel 2 | 3 | 4 | 4 | 3 | 3 | 4 | - | - | - | - |
| Unbehandelt | 0 | 0 | 0 | 0 | 0 | 0 | 0 | 0 | 0 | 0 |

**Beispiel B**

Im Gewächshaus wurden die aufgeführten Pflanzenspezies nach dem Auflaufen mit den aufgeführten Verbindungen in einer Aufwandmenge von 0,1 kg Wirkstoff/ha behandelt. Die Verbindungen wurden zu diesem Zweck als Emulsion mit 500 Litern Wasser/ha gleichmäßig über die Pflanzen versprüht. Hier zeigte zwei Wochen nach der Behandlung die erfindungsgemäße Verbindung eine hohe Kulturpflanzenselektivität in Baumwolle bei ausgezeichneter Wirkung gegen das Unkraut. Das Vergleichsmittel zeigte nicht die gleichhohe Wirksamkeit.
In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung

GOSHI = Gossypium hirsutum
ALOMY = Alopecurus myosuroides
PANSS = Panicum maximum
ABUTH = Abutilon theophrasti
IPOSS = Ipomoea purpurea
POLSS = Polygonum sp.
SOLSS = Solanum sp.
VERPE = Veronica persica
VIOSS = Viola sp.

| Erfindungsgemäße Verbindung | GOSHI | ALOMY | PANSS | ABUTH | IPOSS | POLSS | SOLSS | VERPE | VIOSS |
|---|---|---|---|---|---|---|---|---|---|
| Beispiel 1 | 1 | 3 | 3 | 4 | 3 | 3 | 3 | 3 | 3 |
| Unbehandelt | 0 | 0 | 0 | 0 | 0 | 0 | 0 | 0 | 0 |
| <u>Vergleichsmittel</u> (gemäß EP-A-0 347 811) Verbindung Nr. 243 | 0 | 1 | 1 | 1 | 0 | 1 | 2 | 1 | 0 |

Beispiel C

Im Gewächshaus wurden die aufgeführten Pflanzenspezies nach dem Auflaufen mit den aufgeführten Verbindungen in einer Aufwandmenge von 0,1 kg Wirkstoff/ha behandelt. Die Verbindungen wurden zu diesem Zweck als Emulsion mit 500 Litern Wasser/ha gleichmäßig über die Pflanzen versprüht. Hier zeigte zwei Wochen nach der Behandlung die erfindungsgemäße Verbindung eine hohe Kulturpflanzenselektivität in Baumwolle bei ausgezeichneter Wirkung gegen das Unkraut. Das Vergleichsmittel zeigte nicht die gleichhohe Wirksamkeit.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung

GOSHI = Gossypium hirsutum
AGRRE = Elymus repens
BROTE = Bromus tectorum
SETVI = Setaria viridis
PANSS = Panicum maximum
SORHA = Sorghum halepense
ABUTH = Abutilon theophrasti
GALAP = Galium aparine
IPOSS = Ipomoea purpurea
MATCH = Matricaria chamomilla
POLSS = Polygonum sp.
SEBEX = Sesbania exaltata
VERPE = Veronica persica

| Erfindungsgemäße Verbindung | GOSHI | AGREE | BROTE | SETVI | PANSS | SORHA | ABUTH | GALAP | IPOSS | MATCH | POLSS | SEBEX | VERPE |
|---|---|---|---|---|---|---|---|---|---|---|---|---|---|
| Beispiel 2 | 1 | 3 | 3 | 3 | 3 | 3 | 3 | 3 | 3 | 3 | 4 | 4 | 3 |
| Unbehandelt | 0 | 0 | 0 | 0 | 0 | 0 | 0 | 0 | 0 | 0 | 0 | 0 | 0 |
| Vergleichsmittel | | | | | | | | | | | | | |
| (gemäß EP-A-0 347 811) Verbindung Nr. 4 | 0 | 1 | 0 | 2 | 2 | 2 | 0 | 2 | 0 | 0 | 1 | 2 | 2 |

## Patentansprüche

1. Substituierte α-Pyrimidinyloxy-carbonsäurederivate der allgemeinen Formel I

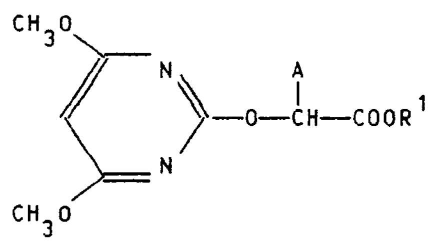

(I)

in der

A einen in 1-Stellung durch Fluor substituierten $C_{1-6}$-Alkyl- oder $C_{3-6}$-Cycloalkylrest oder den α-Methyl-α-fluorbenzylrest und

$R^1$ ein Wasserstoffatom, einen $C_{1-4}$-Alkylrest oder den Benzylrest bedeuten.

2. Verfahren zur Herstellung von Verbindungen der allgemeinen Formel I gemäß Anspruch 1 dadurch gekennzeichnet, daß man

A) Verbindungen der allgemeinen Formel II

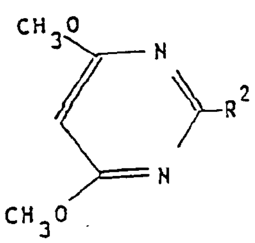

(II)

in der $R^2$ für ein Halogenatom, eine Alkylsulfonylgruppe oder eine Phenylsulfonylgruppe steht, mit Verbindungen der allgemeinen Formel III

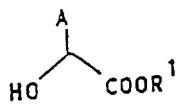

(III) ,

in der A und $R^1$ die in Anspruch 1 genannten Bedeutungen haben, in einem geeigneten Lösungsmittel in Gegenwart einer geeigneten Base umsetzt, oder

B) Verbindungen der allgemeinen Formel IV

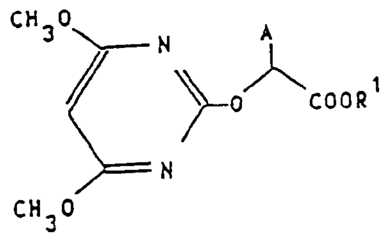

(IV) ,

in der A und $R^1$ in Anspruch 1 angegebenen Bedeutungen haben, in einem geeigneten polaren Lösungsmittel mit einer Alkalimetallbase oder einen Erdalkalimetallbase zu Verbindungen der allgemeinen Formel V

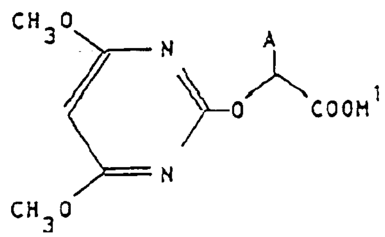

(V) ,

in der A die in Anspruch 1 angegebene Bedeutung hat und $M^1$ für ein Alkalimetallatom oder ein Äquivalent eines Erdalkalimetallatoms steht, umsetzt, und anschließend mit einer geeigneten Säure in einem geeigneten Lösungsmittel zu Verbindungen der allgemeinen Formel VI

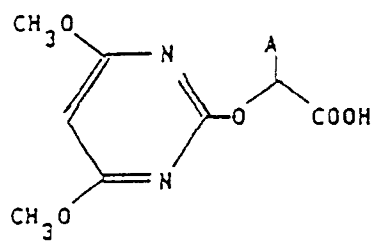

(VI)

in der A die in Anspruch 1 angegebene Bedeutung hat, umsetzt, oder

C) Verbindungen der allgemeinen Formel VIII

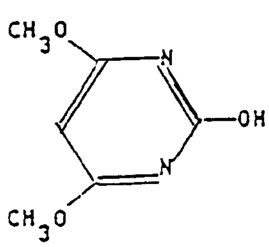

(VIII)

mit einer Verbindung der allgemeinen Formel IX

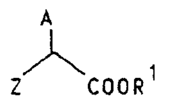

(IX)

in der A und $R^1$ die in Anspruch 1 angegebenen Bedeutungen haben und Z für ein Halogenatom oder eine Alkylsulfonyloxygruppe steht, in Anwesenheit eines geeigneten Lösungsmittels und einer geeigneten Base umsetzt, oder

D) eine Verbindung der allgemeinen Formel X

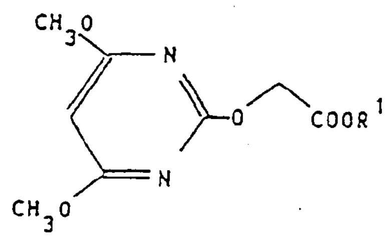

(X)

in der $R^1$ die in Anspruch 1 angegebene Bedeutung hat, mit einer Verbindung der allgemeinen Formel XI

$$A - R^2 \qquad (XI)$$

in der A die in Anspruch 1 angegebene bedeutung hat und $R^2$ für ein Halogenatom, eine Alkylsulfonyloxygruppe oder Phenylsulfonyloxygruppe in einem geeigneten Lösungsmittel in Anwesenheit einer geeigneten Base umsetzt.

3. Mittel mit herbizider, fungizider und pflanzenwachstumsregulierender Wirkung, gekennzeichnet durch einen Gehalt an mindestens einer Verbindung gemäß Anspruch 1.

4. Verwendung von Mitteln gemäß dem Anspruch 3 zur Bekämpfung monokotyler und dikotyler Unkrautarten in landwirtschaftlichen Hauptkulturen.

5. Verwendung von Mitteln gemäß dem Anspruch 3 zur Bekämpfung eines Pilzbefalles in landwirtschaftlichen Kulturen.

6. Verwendung von Mitteln gemäß Anspruch 3 zur Wachstumsbeeinflussung von Kulturpflanzen.

## Claims

1. Substituted α-pyrimidinyloxy(thio)- and α-triazinyloxy(thio)carboxylic acid derivatives of general formula I

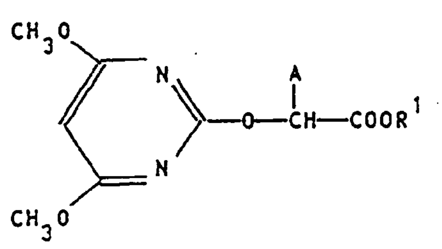

(I)

in which

A is a $C_1$-$C_6$-alkyl or $C_3$-$C_6$-cycloalkyl group substituted in the 1-position by fluorine, or is α-methyl-α-methylbenzyl and

$R^1$ is hydrogen, a $C_1$-$C_4$-alkyl or benzyl group.

2. Process for the preparation of compounds of general formula I, according to claim 1, characterised in that A) compounds of general formula II

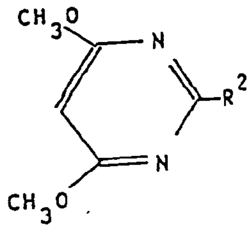

(II)

in which $R^2$ is a halogen atom, an alkylsulfonyl group or a phenylsulfonyl group are reacted with compounds of general formula III

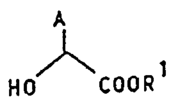

(III)

in which A and $R^1$ have the meanings given under general formula I, in a suitable solvent in the presence of a suitable base, or
B) compounds of general formula IV

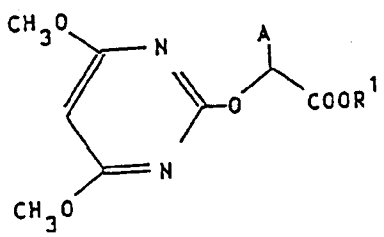

(IV)

in which A and $R^1$ have the meanings given in claim 1 are treated with an alkali metal base or an alkaline earth metal base, in a suitable polar solvent, to give compounds of general formula V

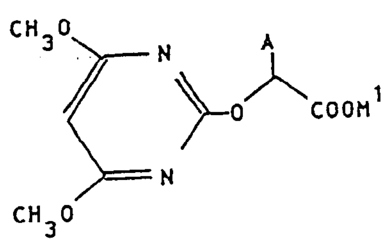

(V)

in which A has the meaning given in claim 1 and $M^1$ is an alkali metal atom or an equivalent of an alkaline earth matal atom, and then with a suitable acid in a suitable solvent to give compounds of general formula VI

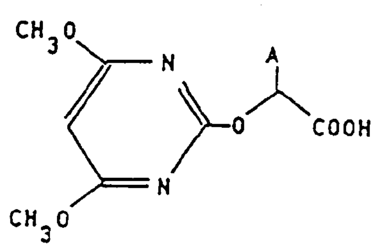

(VI)

in which A has the meaning given in claim 1, or
C) compounds of general formula VIII

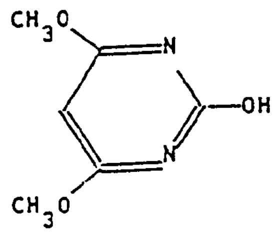

(VIII)

are reacted with a compound of general formula IX

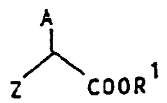

(IX)

in which A and $R^1$ have the meanings given in claim 1 and Z is a halogen atom or an alkylsulfonyloxy group, in the presence of a suitable solvent and a suitable base, or
D) a compound of general formula X

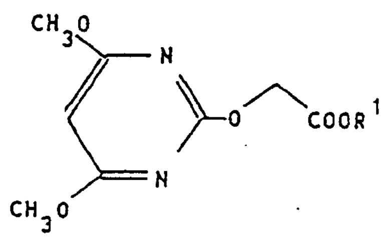

(X)

in which $R^1$ has the meaning given in claim 1 is reacted with a compound of general formula XI

$$A - R^2 \qquad (XI)$$

in which A has the meaning given in claim 1 and $R^2$ is a halogen atom, an alkylsulfonyl group or a phenylsulfonyl group in a suitable solvent in the presence of a suitable base.

**3.** Herbicidal, fungicidal and plant growth regulant compositions characterised by a content of at least one compound according to claim 1.

**4.** Use of the compositions according to claim 3 for the control of monocotyledenous and dicotyledenous weeds in crops.

**5.** Use of the compositions according to claim 3 for the combating fungal attacks in crops.

**6.** Use of the compositions according to claim 3 for the growth regulation of crops.

### Revendications

**1.** Acides α-pyrimidinyloxy-carboxyliques et dérivés de ces adices, de formule génréale I ci-dessous :

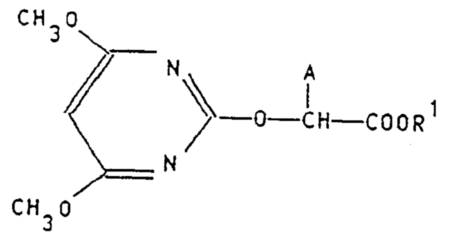

(I)

dans laquelle :

A désigne un alkyle en C1-6 ou un cycloalkyle en C3-6 avec un atome de fluor à la position 1 ou bien le radical α-méthyl-α-fluorobenzyle et

$R^1$ un atome d'hydrogène, un alkyle en C1-4 ou le radical benzyle.

**2.** Procédé de préparation des composés de formule I selon la revendication 1, procédé caractérisé en ce que :

A) On fait réagir des composés de formule générale II :

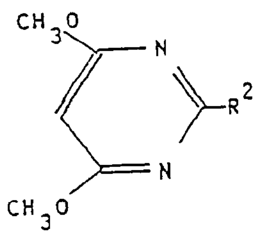

(II)

$R^2$ désignant un atome d'halogène ou un groupe alkylsulfonyle ou phénylsulfonyle, avec des composés de formule générale III :

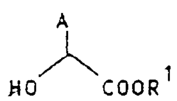

(III)

dans un solvant approprié et en présence d'une base appropriée, ou bien

B) on fait réagir des composés de formule générale IV :

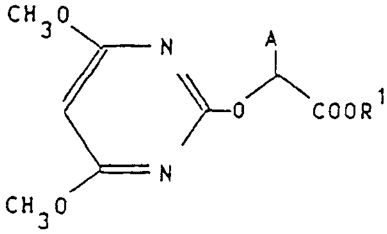

(IV)

dans un solvant polaire approprié, avec une base de métal alcalin ou alcalino-terreux, pour former des composés de formule générale V :

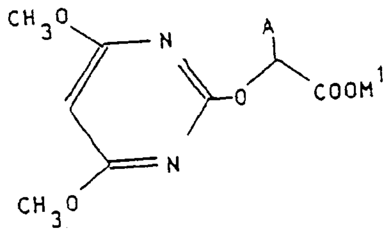

(V)

$M^1$ désignant un atome de métal alcalin ou un équivalent d'un atome de métal alcalino-terreux, composés que l'on transforme ensuite avec un acide approprié, dans un solvant approprié, en composés de formule générale VI :

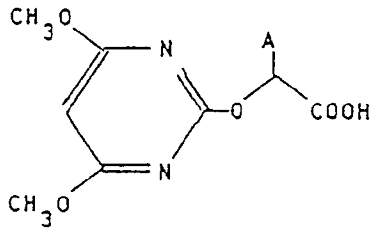

(VI)

ou bien
C) on fait réagir des composés de formule générale VIII :

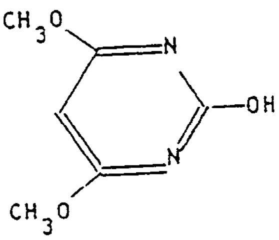

(VIII)

avec un composé de formule générale IX :

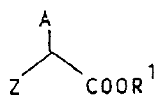

(IX)

Z représentant un atome d'halogène ou un groupe alkylsulfonyloxy, en présence d'un solvant et d'une base appropriés, ou encore
D) on fait réagir un composé de formule générale X :

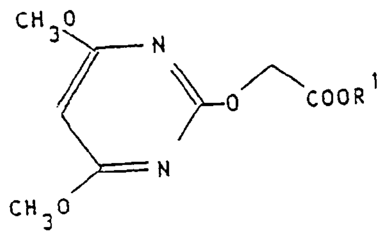

(X)

avec un composé de formule générale XI :

$$A - R^2 \qquad (XI)$$

R2 représentant un atome d'halogène ou un groupe alkylsulfonyloxy ou phénylsulfonyloxy, dans un solvant approprié et en présence d'une base appropriée.

3. Produits herbicides, fongicides et régulateurs de la croissance de végétaux, caractérisés en ce qu'ils contiennent un ou plusieurs composés de la revendication 1.

4. L'emploi des produits de la revendication 3 pour combattre des mauvaises herbes et plantes adventices monocotylédones et dicotylédones en agriculture.

5. L'emploi des produits de la revendication 3 pour combattre des mycoses de plantes en agriculture.

6. L'emploi des produits de la revendication 3 pour agir sur le développement de plantes cultivées.